# EUROPEAN PATENT SPECIFICATION

(11) **EP 3 897 975 B1**
(45) Date of publication and mention of the grant of the patent: **04.11.2026**
(21) Application number: 19817370.0
(22) Date of filing: 16.12.2019
(51) Int. Cl.: B01J 37/00, B01J 37/08, B01J 37/03, B01J 37/04, B01J 21/04, B01J 23/28, B01J 23/00, B01J 27/057, C07C 5/48, C07C 51/25, C07C 51/215, B01J 35/61, B01J 35/63

(54) **CATALYST FOR ALKANE OXIDATIVE DEHYDROGENATION AND/OR ALKENE OXIDATION**
KATALYSATOR ZUR OXIDATIVEN ALKANDEHYDRIERUNG UND/ODER ALKENOXYDIERUNG
CATALYSEUR POUR LA DÉSHYDROGÉNATION OXYDATIVE D'ALCANE ET/OU L'OXYDATION D'ALCÈNE

(30) Priority: 19.12.2018 EP 18213913
(43) Date of publication of application: 27.10.2021
(73) Proprietor: Clariant International Ltd, 4132 Muttenz (CH)
(72) Inventor: SCHRICKER, Ralf, 06237 Leuna (DE); KLEMT, Andreas, 06237 Leuna (DE); STOBBE, Erwin, Roderick, 1031 HW Amsterdam (NL); COLIJN, Hendrik, Albertus, 1031 HW Amsterdam (NL); VAN ROSSUM, Guus, 1031 HW Amsterdam (NL); BOS, Alouisius, Nicolaas, Renée, 1031 HW Amsterdam (NL); SCHOONEBEEK, Ronald, Jan, 1031 HW Amsterdam (NL); SCHUT, Peter, Alexander, 1031 HW Amsterdam (NL); CHRISTIANSEN, Matthew, Adam, Houston, TX 77082-3101 (US)
(74) Representative: Zusammenschluss Clariant
(86) International application number: PCT/EP2019/085290
(87) International publication number: WO 2020/127003

(56) References cited:
- WO-A1-2018/015479
- WO-A1-2018/127767
- WO-A1-2018/141654

## Description

### Field of the invention

The present invention relates to a process for preparing a catalyst for alkane oxidative dehydrogenation (oxydehydrogenation; ODH).

It is known to oxidatively dehydrogenate alkanes, such as alkanes containing 2 to 6 carbon atoms, for example ethane or propane resulting in ethylene and propylene, respectively, in an oxidative dehydrogenation (oxydehydrogenation; ODH) process. Examples of alkane ODH processes, including catalysts and other process conditions, are for example disclosed in US7091377, WO2003064035, US20040147393, WO2010096909 and US20100256432. Mixed metal oxide catalysts containing molybdenum (Mo), vanadium (V), niobium (Nb) and optionally tellurium (Te) as the metals, can be used as such oxydehydrogenation catalysts. Such catalysts may also be used in the direct oxidation of alkenes to carboxylic acids, such as in the oxidation of alkenes containing 2 to 6 carbon atoms, for example ethylene or propylene resulting in acetic acid and acrylic acid, respectively.

Further, WO2018015479 discloses a catalyst preparation process which comprises: 1) mixing a mixed metal oxide (MMO) of molybdenum, vanadium, niobium and optionally tellurium with ceria particles having a crystallite size greater than 15 nm, wherein the amount of the ceria particles, based on the total amount of the catalyst, is of from 1 to 60 wt.%; 2) shaping the mixture thus obtained, which shaping may comprise tableting the mixture or extruding the mixture resulting in tablets or extruded shaped bodies, respectively; and 3) subjecting the tablets or extruded shaped bodies thus obtained to a temperature in the range of from 150 to 500 °C. Further, said WO2018015479 discloses that in addition to said ceria particles, the catalyst may comprise one or more support materials, which may be selected from the group consisting of silica, alumina and silica-alumina. Still further, said WO2018015479 discloses that the weight ratio of said ceria particles to said one or more support materials may vary widely and may be of from 0.1:1 to 20:1, suitably of from 0.1:1 to 10:1, more suitably of from 0.5:1 to 5:1. In the Examples of said WO2018015479, the MMO powder was mixed with silica particles and/or ceria particles, also as powder. No tableting was performed but extrusion followed by calcination.
WO2018127767 discloses oxidative dehydrogenation catalysts comprising MoVNbTeO having certain molar ratios of the metals and exhibiting improved consistency of composition and a 25% conversion of ethylene at less than 420°C and a selectivity to ethylene above 95%, which are prepared by treating the catalyst precursor with H₂O₂ in an amount equivalent to 0.30 - 2.8 mL H₂O₂ of a 30% solution per gram of catalyst precursor prior to calcining and treating the resulting catalyst with the equivalent amount of peroxide after calcining.

It is an object of the present invention to provide a shaped mixed metal oxide catalyst containing Mo, V, Nb and optionally Te which has a relatively high mechanical strength and/or a relatively high activity and/or a relatively high selectivity in the oxidative dehydrogenation of alkanes containing 2 to 6 carbon atoms, for example ethane or propane, and/or in the oxidation of alkenes containing 2 to 6 carbon atoms, for example ethylene or propylene.

### Summary of the invention

Surprisingly it was found that the above-mentioned object may be achieved by means of a process wherein a catalyst containing Mo, V, Nb and optionally Te is mixed with a binder, which binder has a surface area greater than 100 m²/g and a water loss upon heating at a temperature of 485 °C which is greater than 1 wt.%, and subsequently shaped by means of tableting and then heated.

Accordingly, the present invention relates to a process for preparing a shaped catalyst for alkane oxidative dehydrogenation and/or alkene oxidation, which comprises:
a) preparing a mixed metal oxide catalyst containing molybdenum, vanadium, niobium and optionally tellurium;
b) mixing the catalyst obtained in step a), a binder and optionally water, wherein the binder is a hydrated inorganic binder which comprises chemically bonded water and has a Brunauer-Emmett-Teller (BET) has a surface area greater than 100 m²/g and a water loss upon heating at a temperature of 485 °C which is greater than 1 wt.%, wherein said water loss is represented by the difference between the binder weight after heating the binder at a temperature of 110 °C and the binder weight after heating the binder at a temperature of 485 °C, relative to the binder weight after heating the binder at a temperature of 110 °C; and wherein the amount of binder is of from 1 to 70 wt., wherein said amount of binder is the amount of binder, originating from the binder as defined above, in the final catalyst based on the total amount of the final catalyst;
c) shaping the mixture obtained in step b) to form a shaped catalyst by means of tableting; and
d) subjecting the shaped catalyst obtained in step c) to an elevated temperature which is of from 150 to 800 °C.

### Detailed description of the invention

The process of the present invention comprises steps a), b), c) and d), as described hereinbelow. Said process may comprise one or more intermediate steps between steps a) and b), between steps b) and c), and between steps c) and d). Further, said process may comprise one or more additional steps preceding step a) and/or following step d).

While the process of the present invention and gas mixtures or gas streams or catalysts used or produced in said process are described in terms of "comprising", "containing" or "including" one or more various described steps and components, respectively, they can also "consist essentially of" or "consist of" said one or more various described steps and components, respectively.

In the context of the present invention, in a case where a gas mixture or gas stream or a catalyst comprises two or more components, these components are to be selected in an overall amount not to exceed 100%.

Further, where upper and lower limits are quoted for a property then a range of values defined by a combination of any of the upper limits with any of the lower limits is also implied.

In step b) of the shaped catalyst preparation process of the present invention, the mixed metal oxide catalyst containing molybdenum, vanadium, niobium and optionally tellurium obtained in step a), a binder and optionally water are mixed, wherein the binder is a hydrated inorganic binder which comprises chemically bonded water and has a Brunauer-Emmett-Teller (BET)has a surface area greater than 100 m²/g and a water loss upon heating at a temperature of 485 °C which is greater than 1 wt.%.

Surprisingly, it has been found that using the above-described binder in a catalyst shaping process for mixed metal oxide catalysts containing molybdenum, vanadium, niobium and optionally tellurium, advantageously results in both a higher mechanical strength and a higher activity of the final shaped catalyst in alkane oxidative dehydrogenation and alkene oxidation, as further explained in the Examples below.

In the present invention, the binder to be used in step b) has a water loss which is greater than 1 wt.% upon heating at a temperature of 485 °C. Said water loss is represented by the difference between the binder weight after heating the binder at a temperature of 110 °C and the binder weight after heating the binder at a temperature of 485 °C, relative to the binder weight after heating the binder at a temperature of 110 °C. Said water loss may be determined by heating the binder at a temperature of 110 °C for about 4 hours followed by determining the total weight of the binder, and then heating the binder to a temperature of 485 °C followed by heating the binder at a temperature of 485 °C for about 2 hours followed by determining the total weight of the binder. The difference between said two total binder weights, relative to the binder weight after heating the binder at a temperature of 110 °C, represents the water loss (in wt.%) at a temperature of 485 °C.

A clear distinction should be made between "drying" of a binder on the one hand and "dehydration" of a binder on the other hand. The former process only involves the removal of water which is "physically bonded" to the binder. Such water can be removed by evaporating water at for example 100 °C and atmospheric pressure in a dry flow of nitrogen. The other process wherein water is removed ("dehydration"), involves a condensation reaction and takes place at higher temperatures. The water removed in the latter case is normally referred to as "chemically bonded" water. Therefore, the binder to be used in step b) is a hydrated inorganic binder which means that it comprises chemically bonded water.

The above implies that when determining the water loss of the present hydrated binder to be used in step b), first any water physically bonded to the hydrated binder should be removed, for example by drying the hydrated binder at a temperature of for example 100 °C. Then the water loss (loss of chemically bonded water) for the dry (but still hydrated) binder may be determined by heating at a temperature of 485 °C, as described above. In the present invention, the latter water loss should be greater than 1 wt.%, preferably at least 2 wt.%, more preferably at least 3 wt.%, more preferably at least 5 wt.%, more preferably at least 7 wt.%, more preferably at least 10 wt.%, most preferably at least 15 wt.%. Further, in the present invention, the latter water loss may be at most 40 wt.%, preferably at most 35 wt.%, more preferably at most 30 wt.%, more preferably at most 25 wt.%, most preferably at most 20 wt.%. Further, said water loss of the hydrated binder is a property of the binder before it is mixed in step b) with the catalyst obtained in step a).

In the present invention, the hydrated binder should have a surface area greater than 100 m²/g, preferably of from 150 to 500 m²/g, more preferably of from 200 to 450 m²/g, most preferably of from 250 to 400 m²/g. By "surface area", reference is made to the Brunauer-Emmett-Teller (BET) surface area. Further, said surface area of the hydrated binder is the surface area of the binder before it is mixed in step b) with the catalyst obtained in step a).

Further, the hydrated binder preferably has a pore volume of at least 0.2 ml/g, more preferably at least 0.4 ml/g, most preferably at least 0.5 ml/g. Further, the pore volume of the hydrated binder is preferably at most 1.5 ml/g, more preferably at most 1.2 ml/g, most preferably at most 1.0 ml/g. Said pore volume may be determined by water pore volume measurement through incipient wetness impregnation or by nitrogen adsorption measurements at a temperature of 77 °K and a p/p₀ (pressure relative to ambient pressure) of up to 0.995.

In the present invention, the hydrated binder to be used in step b) may be any hydrated inorganic binder which meets the above requirements regarding surface area and water loss. Said hydrated binder may comprise chemically bonded water in an amount of 0.03 to 8 moles of water per mole of binder, more preferably 0.03 to 5 moles, most preferably 0.05 to 3 moles. In the case of hydrated alumina which is of formula Al₂O₃·xH₂O, x in said formula may be of from 0.5 to 8, preferably of from 0.5 to 5, more preferably of from 1 to 3. Further, in the case of hydrated silica which is of formula SiO₂·xH₂O, x in said formula may be of from 0.03 to 1, preferably of from 0.03 to 0.5, more preferably of from 0.05 to 0.2.

In the present invention, the hydrated binder may be selected from the group consisting of hydrated alumina, hydrated silica, hydrated zirconia, hydrated titania and any mixture thereof. Preferably, the hydrated binder comprises hydrated alumina or hydrated silica or a mixture thereof, more preferably hydrated alumina. Preferably, said hydrated binder comprises a hydroxide, suitably an oxide hydroxide, of aluminium, silicon, zirconium or titanium, preferably aluminium or silicon, most preferably aluminium. Suitable examples of hydrated aluminas which may be used as a hydrated binder in step b) of the present process, are pseudoboehmite, boehmite, gibbsite and bayerite. More preferably, pseudoboehmite or boehmite is used, most preferably pseudoboehmite. Boehmite and pseudoboehmite are aluminium oxide hydroxides, i.e. AlO(OH), which are hydrated aluminas of formula Al₂O₃·xH₂O, wherein x = 1 for boehmite and x = 1-2 for pseudoboehmite. Gibbsite and bayerite are aluminium hydroxides, i.e. Al(OH)₃, which are hydrated aluminas of formula Al₂O₃·3H₂O.

The binder to be used in step b) of the present process comprises hydrated binder, as described above. In addition, non-hydrated binder may be used. The non-hydrated binder may be the dehydrated equivalent of the above-described hydrated binder. Examples of suitable non-hydrated binders are non-hydrated alpha-alumina, non-hydrated gamma-alumina, non-hydrated silica, non-hydrated zirconia, non-hydrated titania and any mixture thereof. In case a non-hydrated binder is used, the weight ratio of hydrated binder to non-hydrated binder may be of from 50:1 to 1:50, suitably of from 10:1 to 1:10. However, preferably, the binder to be used in step b) of the present process consists of hydrated binder, as described above.

Further, agents that have a promoting effect on the catalyst obtained in step a) may be mixed with the other components in step b) of the present process. A suitable example of such promoting agent is ceria. Catalysts comprising a) a mixed metal oxide of molybdenum, vanadium, niobium and optionally tellurium and b) ceria particles having a crystallite size greater than 15 nanometers (nm) are disclosed in WO2018015479, the disclosure of which is herein incorporated by reference. The mixture of mixed metal oxide with ceria, as disclosed in said WO2018015479, may be used in step b) of the present process.

In the present invention the amount of hydrated binder may be of from 1 to 70 wt.%, preferably 1 to 60 wt.%, more preferably 1 to 50 wt.%, more preferably 5 to 40 wt.%, most preferably 5 to 30 wt.%. Said amount of hydrated binder is the amount of binder, originating from the hydrated binder, in the final catalyst based on the total amount of the final catalyst, wherein the final catalyst is the shaped catalyst obtained in step d) of the present process. Depending on the desired volumetric activity level, either a relatively low amount of hydrated binder may be used leading to a relatively high volumetric activity or a relatively high amount of hydrated binder may be used leading to a relatively low volumetric activity. A relatively low volumetric activity may be desired in certain cases, as further described in the Examples below.

In step b), the catalyst and binder may be dry mixed in the absence of water or wet mixed in the presence of water. Further, the temperature in step b) may be of from 0 to 50 °C, suitably of from 10 to 40 °C. Most suitably, the temperature in step b) is ambient temperature.

In step c) of the shaped catalyst preparation process of the present invention, the mixture comprising catalyst and binder obtained in step b), is shaped to form a shaped catalyst by means of tableting. Within the present specification, "tableting" refers to a shaping method which does not involve and is not preceded by extrusion. The shaped catalyst obtained in step c) may have any shape, including cylinders, for example hollow cylinders, trilobes and quadrulobes.

It is preferred that prior to step c) the mixture obtained in step b) is dried. Such drying only needs to be carried out in a case where in step b) water has been used resulting in a mixture comprising catalyst, binder and water. Said drying may be carried out at a temperature of from 50 to 150 °C, suitably 80 to 120 °C. Further, tableting may be carried out in any way known to the skilled person. For example, a lubricant for tableting may be added, such as graphite or a stearate salt, for example aluminium distearate.

In step d) of the shaped catalyst preparation process of the present invention, the shaped catalyst obtained in step c) is subjected to an elevated temperature. Preferably, said elevated temperature is of from 150 to 800 °C, more preferably 200 to 600 °C, more preferably 200 to 500 °C, most preferably 300 to 450 °C.

Step d) may be carried out by contacting the shaped catalyst obtained in step c) with oxygen and/or an inert gas at said elevated temperature. The catalyst treatment in step d) may also be referred to as catalyst calcination.

Said inert gas in said calcination step may be selected from the group consisting of the noble gases, nitrogen (N₂) and carbon dioxide (CO₂), preferably from the group consisting of the noble gases and nitrogen (N₂). More preferably, the inert gas is nitrogen or argon, most preferably nitrogen.

Optionally, said inert gas may comprise oxygen in an amount of less than 10,000 parts per million by volume (ppmv), based on the total volume of the gas mixture comprising the inert gas and oxygen. The amount of oxygen may be of from 10 to less than 10,000 ppmv. Preferably, the amount of oxygen is of from 100 to 9,500, more preferably 400 to 9,000, more preferably 600 to 8,500, more preferably 800 to 8,000, most preferably 900 to 7,500 parts per million by volume.

Any source containing oxygen, such as for example air, may be used in said calcination step.

In case oxygen (e.g. air) is used in step d), said elevated temperature is preferably of from 150 to 500 °C, more preferably of from 250 to 500 °C, most preferably 300 to 450 °C. In case an inert gas (e.g. nitrogen) is used in step d), said elevated temperature is preferably of from 150 to 800 °C, more preferably of from 300 to 600 °C.

Step a) of the shaped catalyst preparation process of the present invention comprises preparing a mixed metal oxide catalyst containing molybdenum, vanadium, niobium and optionally tellurium. Said step a) may comprise various steps, including a step a1) which comprises preparing a catalyst precursor containing molybdenum, vanadium, niobium and optionally tellurium. The catalyst precursor obtained in step a1) is a solid. Any known way to prepare such catalyst precursor may be applied. For example, the catalyst precursor may be prepared by a hydrothermal process using a solution or slurry, preferably an aqueous solution or slurry, comprising molybdenum, vanadium, niobium and optionally tellurium or multiple solutions or slurries, preferably aqueous solutions or slurries, comprising one or more of said metals. Alternatively, the catalyst precursor may be prepared by precipitation of one or more solutions, preferably aqueous solutions, comprising molybdenum, vanadium, niobium and optionally tellurium.

The latter precipitation process may comprise:
preparing two solutions, preferably aqueous solutions, one solution comprising molybdenum, vanadium and optionally tellurium, which solution is preferably prepared at slightly elevated temperature, for example 50 to 90 °C, preferably 60 to 80 °C, and another solution comprising niobium, which solution is preferably prepared at about, or slightly above, room temperature, for example 15 to 40 °C, preferably 20 to 35 °C;
combining said two solutions resulting in a precipitate comprising molybdenum, vanadium, niobium and optionally tellurium, which said precipitate may have the appearance of a gel, slurry or dispersion;
recovering the precipitate thus obtained (the catalyst precursor); and
optionally drying the precipitate.

The precipitate thus obtained may be recovered by removing the solvent, preferably water, which can be done by drying, filtration or any other known means for recovery, preferably by drying, for example by evaporation to dryness, for example with the aid of a rotating evaporator, for example at a temperature of from 30 to 70 °C, preferably 40 to 60 °C, or for example by drying in an oven at a temperature of from 60 to 140 °C, or for example by spray drying. The recovered solid may be dried or further dried at a temperature in the range of from 60 to 150 °C, suitably 80 to 130 °C, more suitably 80 to 120 °C.

In above-mentioned step a1), solutions comprising molybdenum, vanadium, niobium and/or optionally tellurium, preferably aqueous solutions, may first be prepared by admixing. The elements Mo, V, Nb and optionally Te can be incorporated into the admixing step as pure metallic elements, as salts, as oxides, as hydroxides, as alkoxides, as acids, or as mixtures of two or more of the above-mentioned forms. As salts, sulfates, nitrates, oxalates, halides, or oxyhalides may be used. For example, the Mo can be incorporated as molybdic acid, ammonium heptamolybdate, molybdenum chlorides, molybdenum acetate, molybdenum ethoxide and/or molybdenum oxides, preferably ammonium heptamolybdate. The V can be incorporated as ammonium vanadate, ammonium metavanadate, vanadium oxide, vanadyl sulfate, vanadyl oxalate, vanadium chloride or vanadyl trichloride, preferably ammonium metavanadate. The Nb can be incorporated as niobium pentoxide, niobium oxalate, ammonium niobate oxalate, niobium chloride or Nb metal, preferably ammonium niobate oxalate. The optional Te can be incorporated as telluric acid, tellurium dioxide, tellurium ethoxide, tellurium chloride and metallic tellurium, preferably telluric acid.

The catalyst precursor obtained in above-mentioned step a1) may be subjected to an elevated temperature, which is preferably of from 150 to 800 °C, preferably by contacting the catalyst precursor with oxygen and/or an inert gas at said elevated temperature, resulting in a mixed metal oxide catalyst containing molybdenum, vanadium, niobium and optionally tellurium. The latter catalyst treatment may also be referred to as catalyst calcination.

Said inert gas in said calcination step may be selected from the group consisting of the noble gases, nitrogen (N₂) and carbon dioxide (CO₂), preferably from the group consisting of the noble gases and nitrogen (N₂). More preferably, the inert gas is nitrogen or argon, most preferably nitrogen.

Optionally, said inert gas may comprise oxygen in an amount of less than 10,000 parts per million by volume (ppmv), based on the total volume of the gas mixture comprising the inert gas and oxygen. The amount of oxygen may be of from 10 to less than 10,000 ppmv. Preferably, the amount of oxygen is of from 100 to 9,500, more preferably 400 to 9,000, more preferably 600 to 8,500, more preferably 800 to 8,000, most preferably 900 to 7,500 parts per million by volume.

Any source containing oxygen, such as for example air, may be used in said calcination step.

Said calcination step may comprise one or more calcination steps. For example, said calcination step may comprise two calcination steps a2) and a3), wherein step a2) comprises contacting the catalyst precursor obtained in step a1) with oxygen (e.g. air) at an elevated temperature and step a3) comprises contacting the catalyst precursor obtained in step a2) with nitrogen at an elevated temperature.

Preferably, in said step a2) the temperature is of from 120 to 500 °C, more preferably 120 to 400 °C, more preferably 150 to 375 °C, most preferably 150 to 350 °C.

Preferably, in step a3) the temperature is of from 300 to 900 °C, preferably 400 to 800 °C, more preferably 500 to 700 °C.

In specific, in step a) of the present process, the catalyst may be prepared by a process as disclosed in WO2018141652, WO2018141653 and WO2018141654, the disclosures of which are herein incorporated by reference.

In the present invention, the catalyst is a mixed metal oxide catalyst containing molybdenum, vanadium, niobium and optionally tellurium as the metals, which catalyst may have the following formula:

MO₁VₐTe_{b}Nb_{c}Oₙ

wherein:
a, b, c and n represent the ratio of the molar amount of the element in question to the molar amount of molybdenum (Mo);
a (for V) is from 0.01 to 1, preferably 0.05 to 0.60, more preferably 0.10 to 0.40, more preferably 0.20 to 0.35, most preferably 0.25 to 0.30;
b (for Te) is either 0 or from >0 to 1, preferably 0.01 to 0.40, more preferably 0.05 to 0.30, more preferably 0.05 to 0.20, most preferably 0.09 to 0.15;
c (for Nb) is from >0 to 1, preferably 0.01 to 0.40, more preferably 0.05 to 0.30, more preferably 0.10 to 0.25, most preferably 0.14 to 0.20; and
n (for O) is a number which is determined by the valency and frequency of elements other than oxygen.

Examples of oxydehydrogenation processes, including catalysts and other process conditions, are for example disclosed in above-mentioned US7091377, WO2003064035, US20040147393, WO2010096909 and US20100256432, the disclosures of which are herein incorporated by reference.

The amount of the catalyst in said process is not essential. Preferably, a catalytically effective amount of the catalyst is used, that is to say an amount sufficient to promote the alkane oxydehydrogenation and/or alkene oxidation reaction. Although a specific quantity of catalyst is not critical to the invention, preference may be expressed for use of the catalyst in such an amount that the gas hourly space velocity (GHSV) is of from 100 to 50,000 hr⁻¹, suitably of from 200 to 20,000 hr⁻¹, more suitably of from 300 to 15,000 hr⁻¹, most suitably of from 500 to 10,000 hr⁻¹.

In the alkane oxidative dehydrogenation process and/or alkene oxidation process, typical reaction pressures are 0.1-20 bara, and typical reaction temperatures are 100-600 °C, suitably 200-500 °C.

In general, the product stream comprises water in addition to the desired product. Water may easily be separated from said product stream, for example by cooling down the product stream from the reaction temperature to a lower temperature, for example room temperature, so that the water condenses and can then be separated from the product stream.

The invention is further illustrated by the following Examples.

### Examples

### 1) Preparation of the mixed metal oxide (MMO) catalyst

A mixed metal oxide (MMO) catalyst containing molybdenum (Mo), vanadium (V), niobium (Nb) and tellurium (Te) was prepared, for which catalyst the molar ratio of said 4 metals was Mo₁V_{0.29}Nb_{0.17}Te_{0.12}, in the following way.

Two solutions were prepared. Solution 1 was obtained by dissolving 15.8 parts by weight (pbw) of ammonium niobate oxalate and 4 pbw of oxalic acid dihydrate in 160 pbw of water at room temperature. Solution 2 was prepared by dissolving 35.6 pbw of ammonium heptamolybdate tetrahydrate, 6.9 pbw of ammonium metavanadate and 5.8 pbw of telluric acid (Te(OH)₆) in 200 pbw of water at 70 °C. 7 pbw of concentrated nitric acid was then added to solution 2.

The 2 solutions were combined, by quickly pouring solution 2 into solution 1 under vigorous stirring in 3 minutes, which yielded an orange gel-like precipitate (suspension) having a temperature of about 45 °C. This suspension was then aged for about 15 minutes. The suspension was then dried by means of spray drying to remove the water, which yielded a dry, fine powder (the catalyst precursor). Said spray drying was carried out by using an air inlet temperature of 350 °C and product outlet temperature of 115°C.

Subsequently, a 500 grams portion of the catalyst precursor was calcined in air in an air-ventilated oven by heating from room temperature to 320 °C at a rate of 100 °C/hour and keeping it at 320 °C for 2 hours.

The cooled catalyst precursor was then removed from the oven and further calcined in a nitrogen (N₂) stream. The catalyst precursor was heated from room temperature to 600 °C at a rate of 100 °C/hour and kept at 600 °C for 2 hours, after which the catalyst was cooled down to room temperature. The flow of the stream in this calcination step was 15 Nl/hr.

### 2) Comparative shaped catalyst A

1 pbw of the MMO catalyst was mixed with 0.25 pbw of ceria (CeO₂) powder, 0.038 pbw of graphite and 0.37 pbw of water at ambient temperature. This mixture was compacted and pre-granulated for 4 minutes in a mixer and dried at 120 °C for 4 hours. The ceria powder had a surface area of 8 m²/g.

The resulting dry material was pressed into tablets having the shape of a hollow cylinder having a height of 5 mm, an external diameter of 6 mm and an internal diameter of 2 mm. The tablets were calcined in air at 300 °C for 2 hours.

The resulting catalyst A tablets have a composition of MMO:CeO₂:graphite of 78%:19%:3% (in wt.%).

### 3) Shaped catalyst B

Shaped catalyst B was made in the same way as comparative shaped catalyst A, with the exception that 1 pbw of the MMO catalyst was mixed with 0.25 pbw of ceria (CeO₂) powder, 0.048 pbw of graphite, 0.45 pbw of water and 0.25 pbw of pseudoboehmite powder.

The pseudoboehmite powder had a water loss of 19 wt.% upon heating at a temperature of 485 °C. Said water loss was determined by heating the pseudoboehmite powder at a temperature of 110 °C for 4 hours followed by determining the total weight of the powder, and then heating the powder to a temperature of 485 °C (at a rate of 5 °C/min) followed by heating at said temperature of 485 °C for 2 hours followed by determining the total weight of the powder. The difference between said two total binder weights represented the water loss at a temperature of 485 °C. Other properties of the pseudoboehmite powder: 1) surface area = 325 m²/g; 2) pore volume = 0.9 ml/g. Said pore volume was determined by water pore volume measurement through incipient wetness impregnation.

The resulting catalyst B tablets have a composition of MMO:CeO₂:alumina:graphite of 67%:17%:13%:3% (in wt.%).

### 4) Shaped catalyst C

Shaped catalyst C was made in the same way as shaped catalyst B, with the exception that 1 pbw of the MMO catalyst was mixed with 0.25 pbw of ceria (CeO₂) powder, 0.064 pbw of graphite, 1.23 pbw of water and 1.22 pbw of pseudoboehmite powder.

The resulting catalyst C tablets have a composition of MMO:CeO₂:alumina:graphite of 45%:11%:41%:3% (in wt.%).

### 5) Testing of physical properties of the shaped catalysts

The strength of the catalyst tablets was determined by a so-called top crushing strength test. A Dillon TC2 Quantrol was used to quantify the force required to crush a tablet using the following method. One tablet was positioned in between two flat plates, with the flat surfaces of the tablet rings facing both flat plates. The flat plates were pushed together and the force required to crush the tablets was recorded. The measurement was repeated at least 10 times and the average force was calculated.

The compacted bulk density (CBD) of the catalyst tablets was determined by placing a weighed amount in a 100 ml cylinder. After vibration to a stable volume, the volume was determined and the weight-to-volume ratio was calculated.

The data for the crush strength and the CBD of shaped catalysts A, B and C are shown in Table 1 below. The results in Table 1 show that the crush strength is advantageously increased by using pseudoboehmite in preparing the shaped catalyst.

**Table 1**

| | Composition (in wt.%) MMO:CeO₂:Al₂O₃:graphite | Crush strength | CBD (kg/l) | MMO (kg/l) |
|---|---|---|---|---|
| A | 78% : 19% : 0% : 3% | 285 N | 1.16 | 0.90 |
| B | 67% : 17% : 13% : 3% | 585 N | 1.04 | 0.70 |
| C | 45% : 11% : 41% : 3% | 793 N | 0.73 | 0.33 |

### 6) Testing of the catalytic performance of the shaped catalysts in ethane oxidative dehydrogenation

The shaped catalysts thus prepared were tested for catalytic performance in oxidative dehydrogenation of ethane. Prior to evaluating the catalytic performance the catalyst tablets were gently crushed and sieved to a mesh fraction of 30-80 mesh.

700 mg of a sieve fraction of the catalyst was loaded in a steel reactor having an internal diameter (ID) of 4 mm. A gas stream comprising 55 vol.% of nitrogen, 32 vol.% of ethane and 13 vol.% of oxygen was passed downflow over the catalyst at a flow rate of 26 Nml/minute, at atmospheric pressure and at a temperature of 360 °C.

The conversion of ethane was calculated from feed and product gas composition which were measured with an online gas chromatograph (GC) equipped with a thermal conductivity detector (TCD). The catalytic performance of the catalysts was measured after a 60 hours equilibration period at 360 °C.

The data on the catalytic performance for shaped catalysts A, B and C are shown in Table 2 below. In Table 2, in addition to the measured conversions for shaped catalysts A, B and C, the following relative activities for shaped catalysts B and C (as compared to shaped catalyst A) are also shown: 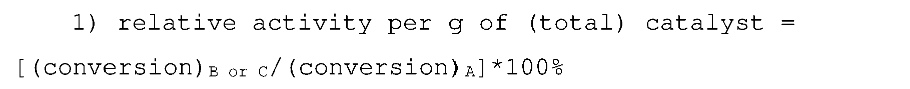 relative activity per g of (total) catalyst = [(conversion)B or C/(conversion)A]*100% 1) 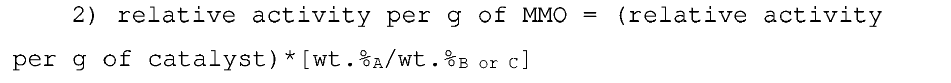 relative activity per g of MMO = (relative activity per g of catalyst) * [wt.%A/wt.%B or C] 2) 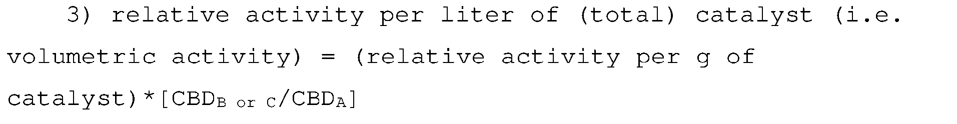 relative activity per liter of (total) catalyst (i.e. volumetric activity) = (relative activity per g of catalyst) * [CBDB or C/CBDA] 3)

**Table 2**

| | Ethane conversion | 1) Relative activity per g of catalyst | 2) Relative activity per g of MMO | 3) Relative activity per liter of catalyst |
|---|---|---|---|---|
| A | 29% | 100% | 100% | 100% |
| B | 34% | 117% | 135% | 104% |
| C | 25% | 86% | 149% | 54% |

The results in Table 2 show that surprisingly by using a hydrated binder (such as pseudoboehmite) in preparing the shaped catalyst, the MMO activity (expressed as activity per g of MMO) is advantageously increased. For example, by using only 13 wt.% of pseudoboehmite (shaped catalyst B), the MMO activity is increased by 35%. Further, using 41% of the hydrated binder (shaped catalyst C) even results in a further increase of the MMO activity, namely by 49%. This is advantageous in that the increase in MMO activity makes it possible to use less of the relatively expensive MMO.

In addition to the above advantageous effect on MMO activity for shaped catalysts B and C, it was observed for shaped catalyst B using 13 wt.% of pseudoboehmite, that surprisingly the lower MMO content and the lower CBD are more than compensated by the above-mentioned increased MMO activity leading to an advantageous increase in volumetric activity of 4%. The volumetrically more active shaped catalyst B has an MMO content of 0.70 kg/l which is lower than that of comparative shaped catalyst A having an MMO content of 0.90 kg/l (see Table 1).

For shaped catalyst C using 41 wt.% of pseudoboehmite instead of 13 wt.% as for shaped catalyst B, a decrease in volumetric activity was observed. A decrease in volumetric activity of 46% was achieved, while at the same time the MMO content was reduced to 0.33 kg/l. Compared to shaped catalyst A, this translates to a 63% reduction of MMO content. However, as already mentioned above, surprisingly, also for shaped catalyst C the MMO activity was advantageously increased.

In some cases, a decrease of volumetric activity, as observed for shaped catalyst C, is not problematic and in combination with the above-described improved MMO activity even advantageous. For there are cases in which one wishes to apply a shaped catalyst having a relatively low volumetric activity, for example if one needs to moderate the volumetric activity in the whole or parts of the reactor wherein a gas stream comprising alkane or alkene and oxygen is passed downflow.

A first example comprises a gradient of volumetric activity or a stacking of discrete volumetric activities in the axial direction of the reactor. Alkane oxidative dehydrogenation and alkene oxidation reactions are highly exothermic while the reaction rate is increasing with increasing partial pressure of the alkane or alkene reactant. As the local heat production at the entrance of the reactor is much higher, this zone of the reactor may be the limiting zone from a heat removal point of view. When distributing the heat removal more evenly over the reactor length, a higher overall heat production and thus higher overall production of desired product(s) can be achieved. Such more even heat removal distribution can be accomplished by loading an increasing volumetric catalyst activity gradient or increasing discrete volumetric catalyst activity levels in the axial direction of the reactor (i.e. "increasing" from entrance to exit of the reactor).

Another example in which moderation of the volumetric activity is attractive is a case where one wishes to operate the reactor at a higher temperature. In the case of ethane oxidative dehydrogenation, it is known that a low temperature favors the formation of acetic acid while a high temperature favors the formation of ethylene. Accordingly, by moderation of the volumetric activity, the temperature can be chosen such as to optimize the product yield distribution between acetic acid and ethylene.

Thus, it is an advantage of the present invention that by adding a hydrated binder not only the MMO activity (expressed as activity per g of MMO) is increased, thus leading to surprisingly lower volumetric MMO contents, but also that the volumetric activity can be fine-tuned to the desired level by varying the amount of the hydrated binder. As the MMO is the most expensive component of the shaped catalyst, efficient use of the MMO is advantageously obtained by the process of the present invention.

## Claims

1. Process for preparing a shaped catalyst for alkane oxidative dehydrogenation and/or alkene oxidation, which comprises:
a) preparing a mixed metal oxide catalyst containing molybdenum, vanadium, niobium and optionally tellurium;
b) mixing the catalyst obtained in step a), a binder and optionally water, wherein the binder is a hydrated inorganic binder which comprises chemically bonded water and has a Brunauer-Emmett-Teller (BET) surface area greater than 100 m²/g and a water loss upon heating at a temperature of 485 °C which is greater than 1 wt.%, wherein said water loss is represented by the difference between the binder weight after heating the binder at a temperature of 110 °C and the binder weight after heating the binder at a temperature of 485 °C, relative to the binder weight after heating the binder at a temperature of 110 °C, and wherein the amount of binder is of from 1 to 70 wt.%, wherein said amount of binder is the amount of binder, originating from the binder as defined above, in the final catalyst based on the total amount of the final catalyst;
c) shaping the mixture obtained in step b) to form a shaped catalyst by means of tableting; and
d) subjecting the shaped catalyst obtained in step c) to an elevated temperature which is of from 150 to 800 °C.

2. Process according to claim 1, wherein the water loss of the binder is at least 2 wt.% and is at most 40 wt.%.

3. Process according to claim 1 or 2, wherein the surface area of the binder is of from 150 to 500 m²/g.

4. Process according to any one of the preceding claims, wherein the binder is selected from the group consisting of hydrated alumina, hydrated silica, hydrated zirconia, hydrated titania and any mixture thereof.

5. Process according to claim 4, wherein the binder comprises hydrated alumina and the hydrated alumina is pseudoboehmite, boehmite, gibbsite or bayerite.

6. Process according to any one of the preceding claims, wherein the amount of binder is of from 1 to 60 wt.%, wherein said amount of binder is the amount of binder, originating from the binder as defined in claim 1, in the final catalyst based on the total amount of the final catalyst.

7. Process according to any one of the preceding claims, wherein the elevated temperature in step d) is of from 200 to 600 °C.

## Patentansprüche

1. Verfahren zur Herstellung eines geformten Katalysators für die oxidative Dehydrierung von Alkanen und/oder die Oxidation von Alkenen, welches umfasst:
a) die Herstellung eines Mischmetalloxid-Katalysators, der Molybdän, Vanadium, Niob und gegebenenfalls Tellur enthält;
b) das Mischen des in Schritt a) erhaltenen Katalysators, eines Bindemittels und gegebenenfalls von Wasser, wobei das Bindemittel ein hydratisiertes anorganisches Bindemittel ist, das chemisch gebundenes Wasser enthält und eine Brunauer-Emmett-Teller (BET)-Oberfläche von mehr als 100 m²/g sowie einen Wasserverlust beim Erhitzen auf eine Temperatur von 485 °C von mehr als 1 Gew.-% aufweist, wobei der genannte Wasserverlust durch die Differenz zwischen dem Gewicht des Bindemittels nach dem Erhitzen auf eine Temperatur von 110 °C und dem Gewicht des Bindemittels nach dem Erhitzen auf eine Temperatur von 485 °C, bezogen auf das Gewicht des Bindemittels nach dem Erhitzen auf eine Temperatur von 110 °C, dargestellt wird und wobei die Menge an Bindemittel zwischen 1 und 70 Gew.-% liegt, wobei die Bindemittelmenge die Menge an Bindemittel ist, die aus dem oben definierten Bindemittel stammt und im Endkatalysator enthalten ist, bezogen auf die Gesamtmenge des Endkatalysators;
c) Formen der in Schritt b) erhaltenen Mischung durch Tablettieren zu einem geformten Katalysator; und
d) den in Schritt c) erhaltenen geformten Katalysator einer erhöhten Temperatur von 150 bis 800 °C aussetzen.

2. Verfahren nach Anspruch 1, wobei der Wasserverlust des Bindemittels mindestens 2 Gew.-% und höchstens 40 Gew.-% beträgt.

3. Verfahren nach Anspruch 1 oder 2, wobei die Oberfläche des Bindemittels zwischen 150 und 500 m²/g beträgt.

4. Verfahren nach einem der vorstehenden Ansprüche, wobei das Bindemittel aus der Gruppe ausgewählt ist, die aus hydratisiertem Aluminiumoxid, hydratisiertem Siliciumdioxid, hydratisiertem Zirkoniumdioxid, hydratisiertem Titandioxid und beliebigen Mischungen davon besteht.

5. Verfahren nach Anspruch 4, wobei das Bindemittel hydratisiertes Aluminiumoxid umfasst und das hydratisierte Aluminiumoxid Pseudoboehmit, Böhmit, Gibbsit oder Bayerit ist.

6. Verfahren nach einem der vorstehenden Ansprüche, wobei die Menge an Bindemittel 1 bis 60 Gew.-% beträgt, wobei die genannte Menge an Bindemittel die Menge an Bindemittel ist, die aus dem in Anspruch 1 definierten Bindemittel stammt, bezogen auf die Gesamtmenge des Endkatalysators.

7. Verfahren nach einem der vorstehenden Ansprüche, wobei die erhöhte Temperatur in Schritt d) zwischen 200 und 600 °C liegt.

## Revendications

1. Procédé de préparation d'un catalyseur moulé pour la déshydrogénation oxydative d'alcanes et/ou l'oxydation d'alcènes, qui comprend :
a) la préparation d'un catalyseur à base d'oxydes métalliques mixtes contenant du molybdène, du vanadium, du niobium et, éventuellement, du tellure ;
b) le mélange du catalyseur obtenu à l'étape a), d'un liant et, éventuellement, d'eau, le liant étant un liant inorganique hydraté qui comprend de l'eau liée chimiquement et qui présente une surface spécifique selon la méthode de Brunauer-Emmett-Teller (BET) supérieure à 100 m²/g et une perte d'eau par chauffage à une température de 485 °C supérieure à 1 % en poids, ladite perte d'eau étant représentée par la différence entre la masse du liant après chauffage à une température de 110 °C et la masse du liant après chauffage à une température de 485 °C, rapportée à la masse du liant après chauffage à une température de 110 °C, et la quantité de liant étant comprise entre 1 et 70 % en masse, dans lequel ladite quantité de liant correspond à la quantité de liant, provenant du liant tel que défini ci-dessus, présente dans le catalyseur final, rapportée à la quantité totale du catalyseur final ;
c) mettre en forme le mélange obtenu à l'étape b) pour former un catalyseur mis en forme par compression en comprimés ; et
d) soumettre le catalyseur moulé obtenu à l'étape c) à une température élevée comprise entre 150 et 800 °C.

2. Procédé selon la revendication 1, dans lequel la perte d'eau du liant est d'au moins 2 % en poids et d'au plus 40 % en poids.

3. Procédé selon la revendication 1 ou 2, dans lequel la surface spécifique du liant est comprise entre 150 et 500 m²/g.

4. Procédé selon l'une quelconque des revendications précédentes, dans lequel le liant est choisi parmi le groupe constitué de l'alumine hydratée, de la silice hydratée, de la zircone hydratée, de la dioxyde de titane hydraté et de tout mélange de ceux-ci.

5. Procédé selon la revendication 4, dans lequel le liant comprend de l'alumine hydratée et ladite alumine hydratée est de la pseudoboehmite, de la boehmite, de la gibbsite ou de la bayerite.

6. Procédé selon l'une quelconque des revendications précédentes, dans lequel la quantité de liant est comprise entre 1 et 60 % en poids, ladite quantité de liant étant la quantité de liant, provenant du liant tel que défini dans la revendication 1, dans le catalyseur final, rapportée à la quantité totale du catalyseur final.

7. Procédé selon l'une quelconque des revendications précédentes, dans lequel la température élevée à l'étape d) est comprise entre 200 et 600 °C.
